# EUROPEAN PATENT APPLICATION

(11) **EP 4 712 089 A1**
(43) Date of publication of application: **18.03.2026**
(21) Application number: 25199163.4
(22) Date of filing: 29.08.2025
(51) Int. Cl.: G16C 20/70, G06F 40/279, G06V 30/413, G06F 16/30, G06F 40/295

(54) **EXTRACTION OF CHEMICAL MOLECULES AND ASSOCIATED PROPERTIES FROM TEXT AND COMPLEX TABLES USING LLMS**

(30) Priority: 17.09.2024 IN 202421070267
(71) Applicant: Tata Consultancy Services Limited, Maharashtra (IN)
(72) Inventor: KRISHNA, Ankur, 411057 Pune, Maharashtra (IN); GADUPARTHI, Trinath, 411028 Pune, Maharashtra (IN); VISHWAKARMA, Arpit, 411057 Pune, Maharashtra (IN)
(74) Representative: Goddar, Heinz J.

(57) **Abstract**

Conventional models extract chemical data through querying tables by decomposing complex user queries. This disclosure relates generally to a method and system for extraction of chemical molecules and associated target properties from text and complex tables using Large Language Models (LLMs). The disclosed method extracts a plurality of molecular property values associated with the chemical molecules, from a plurality data sources, via generating a chemical composition schema utilizing LLMs. The chemical composition schema acts as a lens to view tabular information and a text. Relevant tables are identified and tabular information comprising chemical composition instances are extracted from a plurality of documents utilizing the chemical composition schema, along with LLMs and prompting techniques. The chemical composition instances are curated and reconciled into a unified knowledge graph, which is then used for querying. The disclosed method ensures precision in tabular information extraction without the need for extensive model training.

## Description

### CROSS-REFERENCE TO RELATED APPLICATIONS AND PRIORITY

The present application claims priority to Indian application no. 202421070267, filed on September 17, 2024.

### TECHNICAL FIELD

The disclosure herein generally relates to extraction of chemical molecules, and, more particularly, to a method and system for extraction of chemical molecules and associated properties from text and complex tables using LLMs.

### BACKGROUND

The extraction of chemical molecules and their associated properties is critically important for the development of new compounds or blends that perform better for specific applications such as designing of better refrigerants, design of polymers and composites, designing of new drug compounds, and repurposing newer molecules and blends for newer application Research industries are continually striving to devise technologies to explore the new compounds and the chemical molecules from diverse sources, such as recent publications, patents, and articles. However, much of the information in these documents is presented in complex tables, posing a significant challenge for converting them into query able data. The process of extracting molecular and specific properties from these complex chemical entity tables in research papers and patents is particularly challenging. These tables often come in various formats, such as large, inverted, and multi-column layouts, making automated data extraction difficult. Traditional methods struggle with accurately identifying and interpreting the diverse structures of these tables, resulting in incomplete or erroneous data extraction.

### SUMMARY

Embodiments of the present disclosure present technological improvements as solutions to one or more of the above-mentioned technical problems recognized by the inventors in conventional systems. For example, in one embodiment, a method for extraction of chemical molecules and associated properties from text and complex tables using Large Language Models (LLMs) is provided. The method includes receiving a plurality of documents based on at least one target property of interest amongst a plurality of target properties associated with a plurality of chemical entities, from a plurality of data sources. The plurality of data sources comprises at least one of (i) a plurality of patent documents, (ii) a plurality of technical publications, and (iii) a plurality of chemical databases. Further the method includes identifying a plurality of structured tables with an associated table caption, from each of the plurality of documents by: a) identifying a plurality of table bounds in the plurality of documents using one or more deep learning-based boundary detection techniques, (b) obtaining a table caption for each of the identified plurality of table bounds, and processing the identified plurality of table bounds, using table parsing techniques, to generate the plurality of structured tables with the associated table captions. The method further includes processing the identified plurality of structured tables with the associated table captions along with a plurality of text chunks, using extraction prompts generated via a chemical composition schema, to generate a plurality of chemical composition instances. The method further includes reconciling the plurality of chemical composition instances into a unified knowledge graph.

In another aspect, a system for extraction of chemical molecules and associated properties from text and complex tables using Large Language Models (LLMs) is provided. The system comprising: a memory storing instructions; one or more communication interfaces; and one or more hardware processors coupled to the memory via the one or more communication interfaces, wherein the one or more hardware processors (104) are configured by the instructions to: receive a plurality of documents based on at least one target property of interest amongst a plurality of target properties associated with a plurality of chemical entities, from a plurality of data sources, wherein the plurality of data sources comprises at least one of (i) a plurality of patent documents, (ii) a plurality of technical publications, and (iii) a plurality of chemical databases; identify a plurality of structured tables with an associated table captions, from each of the plurality of documents by: identifying a plurality of table bounds in the plurality of documents using one or more deep learning-based boundary detection techniques; obtaining a table caption for each of the identified plurality of table bounds; and process the identified plurality of table bounds, using table parsing techniques to generate the plurality of structured tables with the associated table caption; process the identified plurality of structured tables with the associated table caption along with a plurality of text chunks, using extraction prompts generated via a chemical composition schema, to generate a plurality of chemical composition instances; and reconcile the plurality of chemical composition instances into a unified knowledge graph.

In yet another aspect, a non-transitory computer readable medium for extraction of chemical molecules and associated properties from text and complex tables using Large Language Models (LLMs) is provided. The method includes receiving a plurality of documents based on at least one target property of interest amongst a plurality of target properties associated with a plurality of chemical entities, from a plurality of data sources. The plurality of data sources comprises at least one of (i) a plurality of patent documents, (ii) a plurality of technical publications, and (iii) a plurality of chemical databases. Further the method includes identifying a plurality of structured tables with an associated table caption, from each of the plurality of documents by: identifying a plurality of table bounds in the plurality of documents using one or more deep learning-based boundary detection techniques, obtaining a table caption for the identified plurality of table bounds, and processing the identified plurality of table bounds, using table parsing techniques to generate the plurality of structured tables with the associated table captions. The method further includes processing the identified plurality of structured tables with the associated table captions along with a plurality of text chunks, using extraction prompts generated via a chemical composition schema, to generate a plurality of chemical composition instances. The method further includes reconciling the plurality of chemical composition instances into a unified knowledge graph.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for extraction of chemical molecules and associated properties from text and complex tables using Large Language Models (LLMs) according to some embodiments of the present disclosure.
FIG. 2 is a functional architecture depicting process flow of the system for extraction of the chemical molecules and the associated properties from the text and the complex tables using the LLMs according to some embodiments of the present disclosure.
FIG. 3 depicts a flow diagram of a method for extraction of the chemical molecules and the associated properties from the text and the complex tables using the LLMs, using the system of FIG. 1, in accordance with some embodiments of the present disclosure.
FIG. 4 depicts obtaining a table caption for each of an identified plurality of table bounds, in accordance with some embodiments of the present disclosure.
FIG. 5 depicts a framework for identifying a plurality of structured tables with associated table captions, from each of a plurality of documents, in accordance with some embodiments of the present disclosure.
FIG. 6 depicts generation of a plurality of chemical composition instances, from the identified plurality of structured tables with the associated table captions along with a plurality of text chunks, using extraction prompts generated via a chemical composition schema, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Existing techniques for chemical data extraction are limited in their ability to accurately and efficiently handle the diverse and complex formats of chemical entity tables found in research publications, patents, and articles. There is a lack of a specialized chemical composition schema tailored for the chemical domain that can address these challenges. Additionally, the existing techniques do not effectively integrate extracted data into a unified knowledge graph or provide seamless natural language query capabilities.

The existing techniques primarily focus on training machine learning models through random frame line manipulation and a weakly supervised learning approach for extracting chemical data. However, these machine learning models need extensive model training to be capable of identifying complex tables.

The complex formats of chemical entity tables in research publications are often complex and varied in format, requiring sophisticated techniques to correctly interpret and extract data. Handling complex formats of chemical entity tables, particularly those spanning multiple pages or columns, necessitates an approach that can scale effectively without loss of accuracy. Extracting meaningful relationships and concepts from the complex formats of chemical entity tables requires deep contextual understanding, which is difficult to achieve with conventional models. Further collating the extracted information from the complex formats of chemical entity tables into a coherent and comprehensive knowledge graph poses significant challenges, particularly in maintaining data consistency and accuracy. Furthermore, developing schema-driven prompts for translating natural language queries into precise knowledge graph queries is a complex task requiring robust language models. These gaps highlight the need for a sophisticated, scalable solution that combines advanced table segmentation techniques, schema driven extraction, and robust language processing capabilities to enhance the accuracy and usability of the extracted molecular property data.

Embodiments herein provide a method and system for extraction of chemical molecules and associated target properties from text and complex tables using Large Language Models (LLMs), in accordance with some embodiments of the present disclosure. The disclosed method extracts a plurality of target molecular property values associated with the chemical molecules, from a plurality data sources, via generating a chemical composition schema utilizing the LLMs. The chemical composition schema acts as a lens to view tabular information and a text. Relevant tables are identified and tabular information comprising chemical composition instances are extracted from documents of a plurality of data sources utilizing the chemical composition schema, along with LLMs and prompting techniques. The chemical composition instances are curated and reconciled into a unified knowledge graph, which is then used for querying. The disclosed method ensures precision in tabular information extraction without the need for extensive model training.

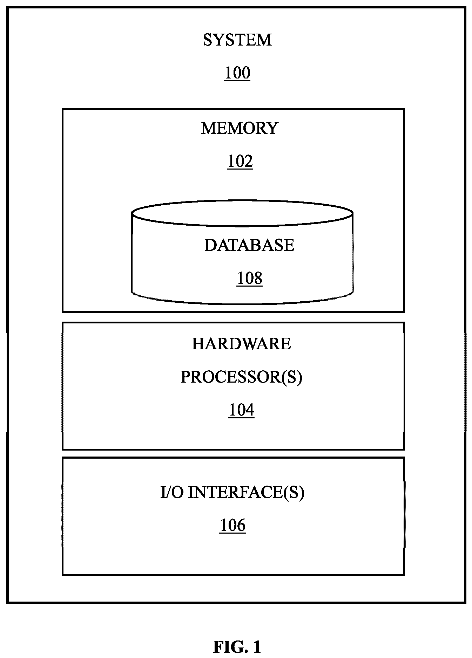

Referring now to the drawings, and more particularly to FIG. 1 through FIG. 6, where similar reference characters denote associated features consistently throughout the figures, there are shown preferred embodiments, and these embodiments are described in the context of the following exemplary system and/or method.

FIG. 1 is a functional block diagram of a system 100 for extraction of chemical molecules and associated properties from text and complex tables using LLMs according to some embodiments of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors.

Referring to the components of the system 100, in an embodiment, the processor (s) 104 can be the one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the processor(s) 104 is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. Thus, the memory 102 may comprise information pertaining to input(s)/output(s) of each step performed by the processor(s) 104 of the system 100 and methods of the present disclosure. In an embodiment, a database 108 is comprised in the memory 102, wherein the database 108 comprises information on a plurality of documents, a plurality of target properties, the plurality of data sources, a plurality of patent documents, a plurality of technical publications, a plurality of chemical databases, the chemical composition schema, a chemical domain guided input prompt, a plurality of structured tables with an associated table caption, a plurality of table bounds, a plurality of chemical composition instances, and the unified knowledge graph, and a user query. The memory 102 further comprises a plurality of modules (not shown) for various technique(s) such as a plurality of Generative Pre-Trained Transformers (GPT) models, one or more deep learning-based boundary detection techniques, a sequential parsing technique, and table parsing techniques. The above-mentioned technique(s) are implemented as at least one of a logically self-contained part of a software program, a self-contained hardware component, and/or, a self-contained hardware component with a logically self-contained part of a software program embedded into each of the hardware component (e.g., hardware processor 104 or memory 102) that when executed perform the method described herein.

The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

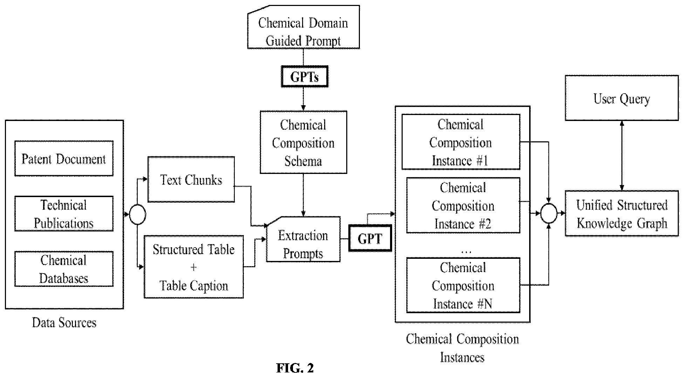

FIG. 2 is a functional architecture depicting process flow of the system 100 of FIG. 1 for extraction of the chemical molecules and the associated properties from the text and the complex tables using LLMs according to some embodiments of the present disclosure. The functional architecture describes the plurality of documents received based on at least one target property of interest amongst the plurality of target properties from the plurality of data sources. Further the plurality of structured tables with the associated table caption, and a plurality of table chunks are identified from the plurality of documents. The identified plurality of structured tables with the associated table caption and the plurality of table chunks are processed using extraction prompts generated via the chemical composition schema, to generate the plurality of chemical composition instances. The generated plurality of chemical composition instances is reconciled into the unified knowledge graph, which can be used for querying by a user.

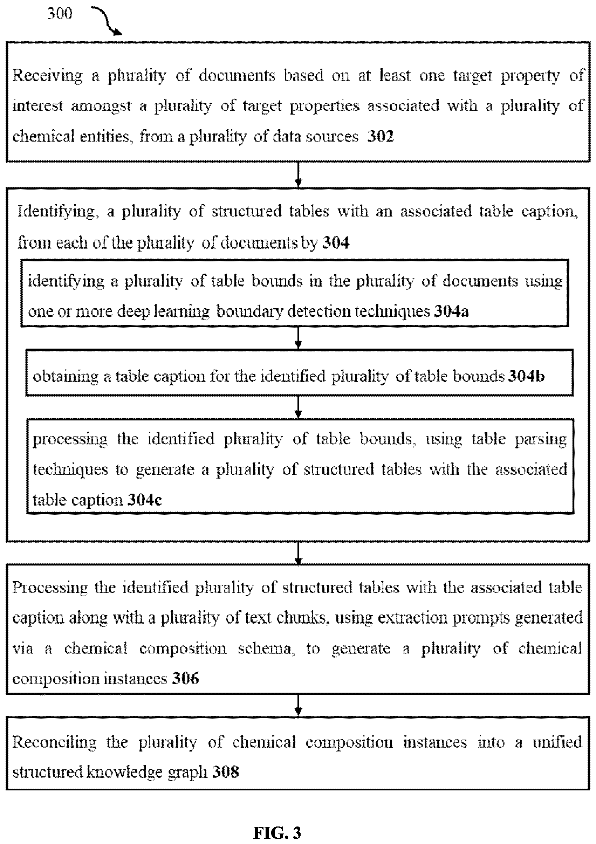

FIG. 3 depicts a flow diagram of a method for extraction of the chemical molecules and the associated properties from the text and the complex tables using LLMs), using the system of FIG. 1, in accordance with some embodiments of the present disclosure.

In an embodiment, the system 100 comprises one or more data storage devices or the memory 102 operatively coupled to the processor(s) 104 and is configured to store instructions for execution of steps of the method 400 by the processor(s) 104. The steps of the method 400 of the present disclosure will now be explained with reference to the components or blocks of the system 100 as depicted in FIG. 1, the functional architecture depicted in FIG. 2, and the steps of flow diagram as depicted in FIG. 3. Although process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

Referring to steps of FIG. 3, at step 302 of the method 300, via one or more hardware processors 104 receive the plurality of documents based on at least one target property of interest amongst the plurality of target properties associated with the plurality of chemical entities, from the plurality of data sources. The chemical entities are distinct chemical substances or molecules, such as atoms, ions, compounds, or mixtures, with specific chemical properties used in various scientific and industrial applications. The plurality of data sources comprises at least one of (i) the plurality of patent documents, (ii) the plurality of technical publications, and (iii) the plurality chemical databases. The plurality of chemical databases comprises Public Chemical Database (PubChem), National Institute of Standards and Technology (NIST) Webbook, Chemical Database of Bioactive Molecules with Drug-Like Properties (ChEMBL), and thereof. The plurality of target properties comprises Normal Boiling Point, Global Warming Potential (GWP), Ozone Depletion Potential, Critical Temperature, Critical Pressure, Critical Volume, Specific Volume, Thermal Conductivity, Vapor Heat Capacity, Solubility in Water, Critical Point Density, Electrical Conductivity, pH Value, Triple Point, Lower Flammability Limit, Occupational Exposure Limit, Molecular Weight, Acentric Factor, Liquid Density, Enthalpy Of Vaporization, Volumetric Capacity, Coefficient of Performance, Lethal Concentration 50 (LC50), and thereof.

While working with problem statements, identifying the target property of interest is crucial for driving the solution process. The plurality of target properties can be derived directly from a problem statement itself received as the user query from the user. With the advent of the LLMs this process is automated and optimized, allowing for extraction of the plurality of target properties more efficiently. For instance, consider the task of designing a refrigerant with a low GWP value. Here, the objective is clearly stated within the problem to minimize the GWP of the refrigerant. In this case, the target property of interest is the GWP value. By leveraging the LLMs, the plurality of target properties from the problem statements can be automatically extracted. The LLM is prompted with a query in the form of the problem statement like like: "Extract the target property of interest from the problem statement design a refrigerant with Low GWP value". The LLM response would identify the target property of interest, which in this case is the GWP value. This automated approach to extracting the plurality of target properties streamlines the process of problem formulation. It enables researchers and engineers to focus on the problem's core objectives while allowing LLMs to handle the task of identifying the plurality of target properties.

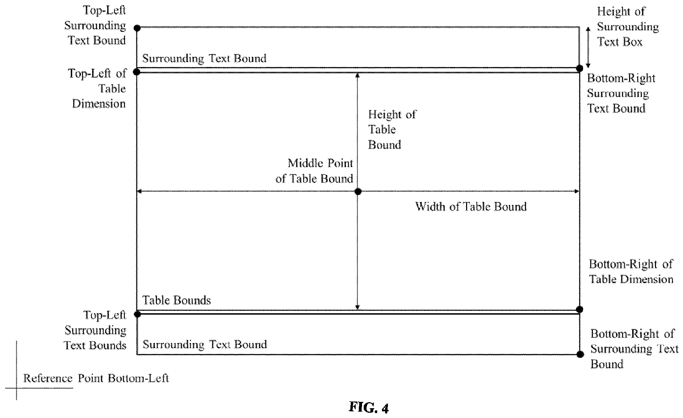

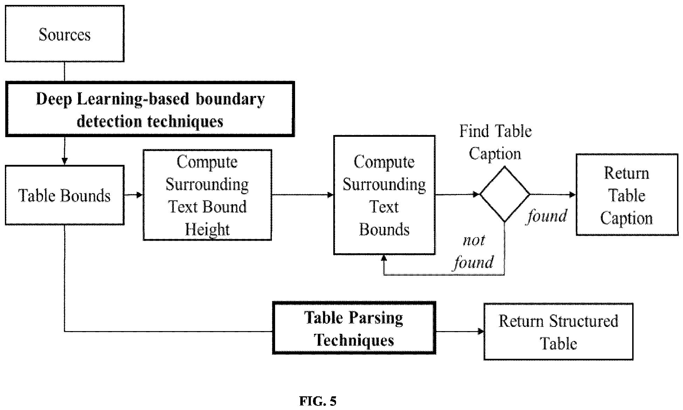

At step 304 of the method 300, the one or more hardware processors 104 identify the plurality of structured tables with the associated table caption, from each of the plurality of documents. FIG. 5 depicts a framework for identifying the plurality of structured tables with the associated table caption, from each of the plurality of documents, in accordance with some embodiments of the present disclosure. The identification of the plurality of structured tables with the associated table caption in FIG.4 is explained through steps 304a through 304c of the method 300. At step 304a, the method 300 identifies the plurality of table bounds in the plurality of documents using one or more deep learning-based boundary detection techniques. The plurality of table bounds in the plurality of documents are identified using a Table Transformer (TATR) model, in accordance with some embodiments of the present disclosure.

Upon identifying the plurality of table bounds in the plurality of documents, the method 300 at step 304b obtains the table caption for the identified plurality of table bounds. A table bound of the plurality of table bounds is represented as [***xₘ, yₘ, wt, ht***]*,* where **(*xₘ*, *yₘ*)** is a middle point of the table bound as per the coordinate system whose origin (reference Point Bottom-Left) is at the bottom-left of the table bound, wt is width of the table bound and *ht* is height of the table bound. The table caption refers to a descriptive label or title given to a table, summarizing the contents or purpose of the table. It helps readers understand the information is being presented in the table and its relevance to the surrounding text or study. FIG. 4 depicts obtaining the table caption for each of the identified plurality of table bounds, in accordance with some embodiments of the present disclosure.

The table caption for each of the identified plurality of table bounds is obtained by:
a) Computing a table dimension for each table bound of the identified plurality of table bounds as **(*t*_{*x*1}*,t*_{*y*1})** and **(*t*_{*x*2}*,t*_{*y*2}).** Here **(*t*_{*x*1}*,t*_{*y*1}) *i***s a top-left point of the table bound and **(*t*_{*x*2}*,t*_{*y*2})** is a bottom-right point of the table bound. ***t*_{*x*1}** is computed as ***xₘ*** - ***wt*/2,** and ***t*_{*y*1}** is computed as ***yₘ*** + ***ht*/*2, t*_{*x*2}** is computed as ***xₘ*** + ***wt*/2,** and ***t*_{*y*2}** is computed as ***yₘ** -* ***ht*/2.**
b) Computing a surrounding text bound height ***sₜₕ*** as n% of height of the table bound ***ht*.** The value of ***n*** as 5 was identified through a heuristic approach, in accordance with some embodiments of the present disclosure. The ***n*** value represents the percentage increase in the height of the table bound that yielded optimal results across the plurality of documents. By expanding the search area by a certain percentage (e.g., 5%,) the table captions are effectively captured that might be slightly misaligned or positioned outside the initially expected area of the table bound.
c) Computing a surrounding text bound comprising a top-left surrounding text bound **(*st*_{*x*1}*,st*_{*y*1})** and a bottom-right surrounding text bound and **(*st*_{*x*2},*st*_{*y*2}),** at top of the table bound, using the table dimension, and the and the surrounding text bound height. Here ***st*_{*x*1} = *t*_{*x*1},*st*_{*y*1} *= t*_{*y*1} *+ s_{th,}, st*_{*x*2} *= t*_{*x*2}, *st*_{*y*2} = *t*_{*y*1}*.***
d) Capturing the surrounding text in the surrounding text bounds, using text parsing technique.
e) Identifying the table caption, by iteratively traversing each line in the surrounding text, using a Regex technique as known in the art. If the table caption is not found at the top of the table bound, the surrounding text bound is recomputed at the bottom of the table bound using the table dimension, and the surrounding text bound height. The table caption is then identified by iteratively traversing each line in the surrounding text, using the Regex technique. Here ***st*_{*x*1} *= t*_{*x*1},*st*_{*y*1} *= t*_{*y*2}*, st*_{*x*2} = *t*_{*x*2}*, st*_{*y*2} = *t*_{*y*1} - *sₜₕ.***

Upon obtaining the table caption for the identified plurality of table bound at step 304c of the method 300 processes the identified plurality of table bounds, using the table parsing techniques to generate the plurality of structured tables with the associated table caption. The identified plurality of table bounds is processed using one of the Camelot table parsing technique and Open Source Computer Vision (OpenCV) Library to generate the plurality of structured tables with the associated table caption, in accordance with some embodiments of the present disclosure.

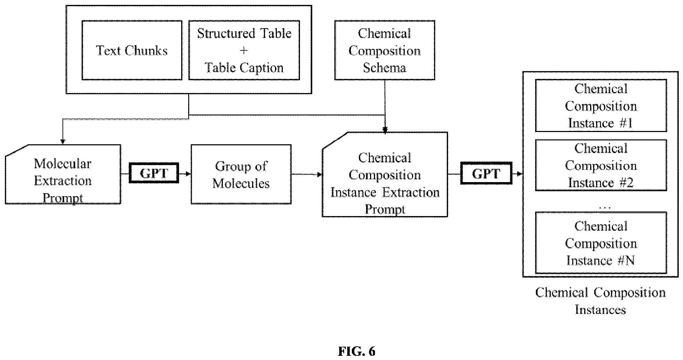

At step 306 of the method 300, the one or more hardware processors 104 process the identified plurality of structured tables with the associated table caption along with a plurality of text chunks, using the extraction prompts generated via the chemical composition schema, to generate the plurality of chemical composition instances. FIG. 6 depicts generation of a plurality of chemical composition instances, from the identified plurality of structured tables with the associated table caption along with the plurality of text chunks, using the extraction prompts generated via the chemical composition schema, in accordance with some embodiments of the present disclosure.

The chemical composition schema is a structured representation of the components that make up a chemical entity. The chemical composition schema includes detailed information about the different constituents of the chemical entity, their types, and their contributions to the overall composition. The chemical composition schema helps in organizing and understanding chemical makeup of substances, serving as a foundation for analyzing and designing chemical processes, formulations, and products.

In the field of a chemical composition, the chemical composition schema would represent the various constituents, such as atoms, molecules, and chemical substances, and their specific contributions in terms of weight percentage (wt.%) as well as its properties. It aids in accurately describing mixtures and blends, composed of more than two different molecules. The scope of the chemical composition schema can be tailored to the specific needs of a problem at hand. It can be curated by chemical experts or the GPTs with appropriate prompts. This structured approach ensures a comprehensive and precise representation of the plurality of chemical entities and their compositions. The disclosed method 300 generates the chemical composition schema through prompting one of the plurality of GPT models using the chemical domain guided input prompt. The chemical domain guided input prompt instructs one of the plurality of GPT models for generating chemical composition schema. The chemical domain guided input prompt provided to one of the plurality of GPT models for generating the chemical composition schema is given by: chemical domain guided input prompt - "Generate a JavaScript Object Notation (JSON) schema that represents "chemical entities" and its relationships including detailed information about their chemical constituents & contributions, its properties, synonyms, **and** structure:", in accordance with some embodiments of the present disclosure.

A partial chemical composition schema generated by one of the plurality of GPT models, in accordance with some embodiments of the present disclosure is as below:

```
 {
 "ChemicalEntities": [
 {
 "Name": "",
 "ChemicalConstituents": [
 {
 "Name": "",
 "Type": "",
 "Contribution": "wt%"
 }
 ],
 "Properties": [
 {
 "PropertyName": "NormalBoilingPoint",
 "PropertyValue": ["value", "Unit"],
 "PropertyClass": "Physical",
 "InformationSource": "",
 "PageNumber": ""
 },
```

The generated chemical composition schema is postprocessed based on the at least one target property of interest among the plurality of target properties. Postprocessing is a critical step to ensure that the plurality of target properties is accurately captured from the given problem statement. For instance, if the objective is to design a molecule with a low GWP value, the chemical composition schema must include the property "GWP".

The extraction prompts are generated via the chemical composition schema comprises the molecular extraction prompt and the composition instance extraction prompt. The extraction prompts are generated using a predefined template, the chemical composition schema, and a text chunk. By combining these three elements, a unique prompt is created for each extraction.

Upon generation of the chemical composition schema and the extraction prompts, the method 300 generates the plurality of chemical composition instances from the identified plurality of structured tables with the associated table caption along with the plurality of text chunks, using the extraction prompts generated via the chemical composition schema. The plurality of text chunks is obtained by reading a text content from the plurality of documents ignoring text in the plurality of table bounds and chunking the text content sequentially into smaller tokens based on input model context length. The plurality of structured tables with the associated table caption, the plurality of text chunks from the plurality of documents, the molecular extraction prompt, is fed to one of the plurality of GPT models. The molecular extraction prompt provided to the one of the plurality of GPT model with respect to the plurality of text chunks for text processing, in accordance with some embodiments of the present disclosure, is defined as: molecular extraction prompt = "Assume you are a chemical engineer. Given a text of compounds and blends delimited by double backticks and the schema model delimited by three backticks: Provide a JSON array containing only the names of the compounds and blends listed in the table. Do not provide any preamble. The array should contain only the names of the compounds and blends present in the text.
Text: ʺ{Text}ʺ
Schema: ‴{schema model}‴"

The molecular extraction prompt provided to the one of the plurality of GPT model is with respect to the plurality of structured tables for table processing, in accordance with some embodiments of the present disclosure, is defined as: molecular extraction prompt = "Assume you are a chemical engineer. Given a table of compounds and blends delimited by double backticks and the schema model delimited by three backticks: Provide a JSON array containing only the names of the compounds and blends listed in the table. Do not provide any preamble. The array should contain the properties mentioned in the given schema.
Table: ʺ{Table}ʺ
Schema: ‴{schema model}‴"

The one of the plurality of GPT model based on the molecular extraction prompt, identifies the plurality of molecules or blends present in the plurality of structured tables with table captions and the plurality of text chunks. The output of the GPT model which is the plurality of molecules present in the plurality of structured tables, in accordance with some embodiments of the present disclosure, is represented as: [ "R1234ze(E) + R152a", "R600a + R1234ze(Z)", "R600a + R1233zd(E)", "R450A (R1234ze(E) + R134a)", "R513A (R1234yf + R134a)", "R1234ze + R134a", "R430A (R152a + R600a)", "R431A (R290 + R152a)", "R435A (RE170 + R152a)", "R436A (R290 + R600a)", "R600a + R1224yd(Z)", "R1243zf + R152a", "R600a + R236ea", "R1234yf + R152a", "R600a + R245fa", "R152a + R600", "R454A (R32 + R1234yf)", "R454C (R32 + R1234yf)", "R32 + R1234yf + R744", "R455A (R744 + R32 + R1234yf)", "R457A (R32 + R1234yf + R152a)", "R459B (R32 + R1234yf + R1234ze(E))"].

Upon identifying the plurality of molecules present in the plurality of structured tables and in the plurality of text chunks, the sequential parsing technique is applied on the identified the plurality of molecules to generate the plurality of groups of molecules. The sequential parsing technique improves accuracy and reduces hallucination of the GPT model. The plurality of molecules is divided into five groups of molecules, in accordance with some embodiments of the present disclosure, is as follows:

```
       Molecule group 1
       [["R1234ze(E) + R152a"],
        ["R600a + R1234ze(Z)"],
        ["R600a + R1233zd(E)"],
        ["R450A (R1234ze(E) + R134a)"],
        ["R513A (R1234yf + R134a)"]
       ]
       Molecule group 2
       [["R1234ze + R134a"],
        ["R430A (R152a + R600a)"],
        ["R431A (R290 + R152a)"],
        ["R435A (RE170 + R152a)"],
        ["R436A (R290 + R600a)"]
        ]
       Molecule group 3
       [["R600a + R1224yd(Z)"],
        ["R1243zf + R152a"],
        ["R600a + R236ea"],
        ["R1234yf + R152a"],
        ["R600a + R245fa"]
       ]
       Molecule group 4
       [["R152a + R600"],
        ["R454A (R32 + R1234yf)"],
        ["R454C (R32 + R1234yf)"],
        ["R32 + R1234yf + R744"],
        ["R455A (R744 + R32 + R1234yf)"]
        ]
       Molecule group 5
       [["R457A (R32 + R1234yf + R152a)"],
        ["R459B (R32 + R1234yf + R1234ze(E))"]
        ]
```

Upon generating the plurality of groups of molecules, the method 300 sequentially feeds each group amongst the plurality of groups of molecules, the plurality of structured tables with table captions, the plurality of text chunks, the chemical composition schema, and the composition instance extraction prompt, to one of the plurality of GPT models, to generate the plurality of chemical composition instances with the plurality of target molecular property values associated with the plurality of target properties. The composition instance extraction prompt provided to one of the plurality of GPT models with respect to the plurality of text chunks for text processing, in accordance with some embodiments of the present disclosure, is defined as: "Assume you are a chemical engineer. Given a text chunk delimited by three backticks, and the schema delimited by double backticks: Extract information for {MoleculeGroup-N} using the Schema from the provided text and generate a structured JSON output for all information mentioned in the text. Do not include any preamble, and only output JSON where information is available. Do not include JSON elements where the value is empty, null, or 0. Treat spaces in values as decimal points, such that '38 2' becomes '38.2'. Do not indent the JSON output.
Schema: ʺ{ schema model}ʺ
Text: ‴{text}‴"

The composition instance extraction prompt provided to the one of the plurality of GPT model is with respect to the plurality of structured tables for table processing, in accordance with some embodiments of the present disclosure, is defined as: composition instance extraction prompt = "Assume you are a chemical engineer. Given a table delimited by three backticks, and the schema delimited by double backticks:Extract information for {MoleculeGroup-N} using the Schema from the provided table and generate a JSON structure for all information mentioned in the table. Do not provide any preamble. Only generate JSON output for which information is available, and do not include JSON elements where the value is empty, null, or Consider spaces in values as decimal points, such as '38 2' being '38.2'. Do not indent the JSON output. Schema: ʺ {schema model} ʺ
Table: ‴{table}‴"

The one of the plurality of GPT models, upon receiving each group amongst the plurality of groups of molecules, the plurality of structured tables with table captions, the plurality of text chunks, the chemical composition schema, the composition instance extraction prompt, generates the plurality of chemical composition instances with the plurality of target molecular property values associated with the plurality of target properties. The GPT model generates the plurality of chemical composition instances with the plurality of target molecular property values associated with the plurality of target properties, in accordance with some embodiments of the present disclosure, is obtained as follows:

```
       {
       "ChemicalEntities": [
       {
       "Name": "R1234ze(E) + R152a",
       "ChemicalConstituents": [
       {
       "Name": "R1234ze(E)",
       "Type": "Molecule",
       "Contribution": "10 wt%"
       },
       {
       "Name": "R152a",
       "Type": "Molecule",
       "Contribution": "90 wt%"
       }
       ],
       "Properties": [
       {
       "PropertyName": "NormalBoilingPoint",
       "PropertyValue": ["-24.41", "C"],
       "PropertyClass": "Physical",
       "InformationSource": "[29,30]",
       "PageNumber": "3"
       },
       {
       "PropertyName": "GlobalWarmingPotential",
       "PropertyValue": ["112.2", "Unit"],
       "PropertyClass": "Environmental",
       "InformationSource": "[29,30]",
       "PageNumber": "3"
       }
       ],
       "Synonyms": [
       {
       "ASHRAE_Designation": "A2"
       }
       ]
       },
       {
       "Name": "R600a + R1234ze(Z)",
       "ChemicalConstituents": [
       {
       "Name": "R600a",
       "Type": "Molecule",
       "Contribution": "90 wt%"
       },
       {
       "Name": "R1234ze(Z)",
       "Type": "Molecule",
       "Contribution": "10 wt%"
       }
       ],
       "Properties": [
       {
       "PropertyName": "NormalBoilingPoint",
       "PropertyValue": ["-11.77", "C"],
       "PropertyClass": "Physical",
       "InformationSource": "[29]",
       "PageNumber": "3"
       },
       {
       "PropertyName": "GlobalWarmingPotential",
       "PropertyValue": ["18.6", "Unit"],
       "PropertyClass": "Environmental",
       "InformationSource": "[29]",
       "PageNumber": "3"
       }
       ],
       "Synonyms": [
       {
       "ASHRAE_Designation": "A3"
       }
       ]
       },
       {
       "Name": "R600a + R1233zd(E)",
       "ChemicalConstituents": [
       {
       "Name": "R600a",
       "Type": "Molecule",
       "Contribution": "90 wt%"
       },
       {
       "Name": "R1233zd(E)",
       "Type": "Molecule",
 "Contribution": "10 wt%"
       }
       ],
       "Properties": [
       {
       "PropertyName": "NormalBoilingPoint",
       "PropertyValue": ["-11.92", "C"],
       "PropertyClass": "Physical",
       "Information Source": "[29]",
       "PageNumber": "3"
       },
       {
       "PropertyName": "GlobalWarmingPotential",
       "PropertyValue": ["31.4", "Unit"],
       "PropertyClass": "Environmental",
       "Information Source": "[29]",
       "PageNumber": "3"
       }
       ],
       "Synonyms": [
       {
       "ASHRAE_Designation": "A3"
       }
       ]
       },
       {
       "Name": "R450A (R1234ze(E) + R134a)",
       "ChemicalConstituents": [
       {
       "Name": "R1234ze(E)",
       "Type": "Molecule",
       "Contribution": "58 wt%"
       },
       {
       "Name": "R134a",
       "Type": "Molecule",
       "Contribution": "42 wt%"
       }
       ],
       "Properties": [
       {
       "PropertyName": "NormalBoilingPoint",
       "PropertyValue": ["-23.36", "C"],
       "PropertyClass": "Physical",
       "InformationSource": "[29,31-33]",
       "PageNumber": "3"
       },
       {
       "PropertyName": "GlobalWarmingPotential",
       "PropertyValue": ["547", "Unit"],
       "PropertyClass": "Environmental",
       "InformationSource": "[29,31-33]",
       "PageNumber": "3"
       }
       ],
       "Synonyms": [
       {
       "ASHRAE_Designation": "A1"
       }
       ]
       },
       {
       "Name": "R513A (R1234yf + R134a)",
       "ChemicalConstituents": [
       {
       "Name": "R1234yf",
       "Type": "Molecule",
       "Contribution": "56 wt%"
       },
       {
       "Name": "R134a",
       "Type": "Molecule",
       "Contribution": "44 wt%"
       }
       ],
       "Properties": [
       {
       "PropertyName": "NormalBoilingPoint",
       "PropertyValue": ["-29.58", "C"],
       "PropertyClass": "Physical",
       "InformationSource": "[31]",
       "PageNumber": "3"
       },
       {
       "PropertyName": "GlobalWarmingPotential",
       "PropertyValue": ["573", "Unit"],
       "PropertyClass": "Environmental",
       "InformationSource": "[31]",
       "PageNumber": "3"
       }
       ],
       "Synonyms": [
       {
       "ASHRAE_Designation": "A1"
       }
       ]
       }
       ]
       }
```

At step 308 of the method 300, the one or more hardware processors 104 reconcile the plurality of chemical composition instances into a unified knowledge graph. The unified knowledge graph provides one or more chemical composition instances among the plurality of chemical composition instances based on a received user query.

### Experimental details

The disclosed method is validated using an extensive dataset comprising the plurality of technical publications, the plurality of patent documents. The objective was to extract the plurality of molecules and blends from the latest technical papers and patent documents for a unique refrigerant that possesses optimal properties, such as low GWP and an optimal Normal Boiling Point (NBP). Chemical composition includes molecular entities, their compositions, and 32 target properties. For validation purposes, publicly available data was downloaded from the Web, comprising 12 technical publications and 1 patent document as shown in Table. 1 and Table. 2 which were processed to extract the plurality of tables and the plurality of text chunks.

**Table. 1**

| **S.No** | **Paper** | **No. of Pages** | **No. of Property Tables** |
|---|---|---|---|
| **P1** | Refrigerant Data Summary, Calm and Hourahan, 2001 | 8 | 2 large tables |
| **P2** | Thermodynamic properties of CFC alternatives: A survey of the available data, McLinden | 14 | 5 |
| **P3** | A thermodynamic analysis of refrigerants: Possibilities and tradeoffs for Low-GWP refrigerants, McLinden et al, 2014 | 13 | 1 large table |
| **P4** | Theoretical analysis of suitable fluids for high temperature heat pumps up to 125 °C heat delivery | 11 | 3 |
| **P5** | Evaluation of flammability characteristics of mixed refrigerants R134a/R1270, R134a/R170, and R170/R1270, Zhang et al, 2023 | 11 | 3 |
| **P6** | Analysis of thermophysical properties and performance of nano refrigerants and nano lubricant-refrigerant mixtures in refrigeration systems, Said et al, 2023 | 10 | 2 |
| **P7** | Investigation and Analysis of R463A as an Alternative Refrigerant to R404A with Lower GWP, Saengsikhiao et al, 2020 | 19 | 8 |
| **P8** | Current status of refrigerants used in domestic applications: A review, Nagarjuna et al, 2023 | 19 | 3 |
| **P9** | Hunting sustainable refrigerants fulfilling technical, environmental, safety and economic requirements, Alba et al, 2023 | 19 | 6 |
| **P10** | Low GWP halocarbon refrigerants: A review of thermophysical properties, Bobbo et al, 2018 | 21 | 12 |
| **P11** | Refrigerant Data Update, Calm and Hourahan, 2007 | 15 | 2 large tables |
| **P12** | Performance comparison of pure, binary and ternary refrigerants considering different systems, Zhao et al, 2023 | 20 | 7 |

**Table. 2**

| **S.No** | **Patent** | **No. of Pages** | **No. of Property Tables** |
|---|---|---|---|
| | | | |
| **PT1** | GASEOUS DIELECTRICS WITH LOW GLOBAL WARMING POTENTIALS, US 8,080,185 B2 | 19 | 2 large tables |

The disclosed method extracted 1,158 molecules with plurality of the target properties. Out of these, 844 molecules (refrigerants) were extracted from the plurality of structured tables, and 187 unique refrigerants were extracted from the plurality of text chunks. The extraction results were manually validated by subject matter experts, achieving an accuracy of 98.7% and a recall of 94%. The F1 score for the extraction process was estimated to be 96.3%.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of automated data extraction from large, inverted, and multi-column layout tables. The embodiment thus provides a method and system for extraction of chemical molecules and associated target properties from text and complex tables using the LLMs. The disclosed method extracts the plurality of molecular property values associated with the chemical molecules, from the plurality data sources, via generating the chemical composition schema utilizing the LLMs. The chemical composition schema acts as a lens to view tabular information and the text. Relevant tables are identified and tabular information comprising chemical composition instances are extracted from a plurality of documents utilizing the chemical composition schema, along with LLMs and prompting techniques. The chemical composition instances are curated and reconciled into a unified knowledge graph, which is then used for querying. The disclosed method ensures precision in tabular information extraction without the need for extensive model training. The disclosed method enables deep chemical knowledge exploration from siloed sources and reconciliation of chemical properties fragmented across multiple data sources.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300), the method comprising:
receiving (302), via one or more hardware processors, a plurality of documents based on at least one target property of interest amongst a plurality of target properties associated with a plurality of chemical entities, from a plurality of data sources, wherein the plurality of data sources comprises at least one of (i) a plurality of patent documents, (ii) a plurality of technical publications, and (iii) a plurality of chemical databases;
identifying (304), via one or more hardware processors, a plurality of structured tables with associated table captions, from each of the plurality of documents by:
a) identifying (304a) a plurality of table bounds in the plurality of documents using one or more deep learning-based boundary detection techniques;
b) obtaining (304b) a table caption for each of the identified plurality of table bounds; and
c) processing (304c) the identified plurality of table bounds, using table parsing techniques, to generate the plurality of structured tables with the associated table captions;
processing (306), via one or more hardware processors, the identified plurality of structured tables with the associated table captions along with a plurality of text chunks, using extraction prompts generated via a chemical composition schema, to generate a plurality of chemical composition instances; and
reconciling (308), via one or more hardware processors, the plurality of chemical composition instances into a unified knowledge graph.

2. The processor implemented method as claimed in claim 1, the unified knowledge graph provides one or more chemical composition instances among the plurality of chemical composition instances based on a received user query.

3. The processor implemented method as claimed in claim 1, wherein the table caption for each of the identified plurality of table bounds is obtained by:
a) computing a table dimension further comprising a top-left table dimension and a bottom-right table dimension for each of the table bound of the identified plurality of table bounds, using a table height of the table bound, a table width of the table bound, and a middle point of the table bound;
b) computing a surrounding text bound height as n% of the height of the table bound;.
c) computing a surrounding text bound further comprising a top-left surrounding text bound and a bottom-right surrounding text bound, at top of the table bound, using the table dimension, and the surrounding text bound height;
d) capturing a surrounding text in the surrounding text bounds, using a text parsing technique; and
e) identifying the table caption, by iteratively traversing each line in the surrounding text, using a Regex technique, wherein if the table caption is not found at the top of the table bound, the surrounding text bound is recomputed at the bottom of the table bound using the table dimension, and the surrounding text bound height, and wherein the table caption is identified by iteratively traversing each line in the surrounding text, using the Regex technique.

4. The processor implemented method as claimed in claim 1, wherein the extraction prompts generated using the chemical composition schema comprises the molecular extraction prompt and the composition instance extraction prompt.

5. The processor implemented method as claimed in claim 1, wherein the chemical composition schema is generated through prompting one of a plurality of Generative Pre-Trained Transformers (GPT) models using a chemical domain guided input prompt, and wherein the generated chemical composition schema is postprocessed based on the at least one target property of interest among the plurality of target properties.

6. The processor implemented method as claimed in claim 1, the plurality of chemical composition instances is generated by:
a) feeding the plurality of structured tables with the associated table captions, the plurality of text chunks from the plurality of documents, a molecular extraction prompt, to one of the plurality of GPT models, wherein the one of the plurality of GPT model identifies a plurality of molecules present in the plurality of structured tables with table captions and in the plurality of text chunks;
b) applying a sequential parsing technique on the identified plurality of molecules to generate a plurality of groups of molecules; and
c) sequentially feeding each group amongst the plurality of groups of molecules, the plurality of structured tables with table captions, the plurality of text chunks, the chemical composition schema, and the composition instance extraction prompt, to one of the plurality of GPT models, to generate the plurality of chemical composition instances with a plurality of target molecular property values associated with the plurality of target properties.

7. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive a plurality of documents based on at least one target property of interest amongst a plurality of target properties associated with a plurality of chemical entities, from a plurality of data sources, wherein the plurality of data sources comprises at least one of (i) a plurality of patent documents, (ii) a plurality of technical publications, and (iii) a plurality of chemical databases;
identify a plurality of structured tables with associated table captions, from each of the plurality of documents by:
a) identifying a plurality of table bounds in the plurality of documents using one or more deep learning-based boundary detection techniques;
b) obtaining a table caption for each of the identified plurality of table bounds; and
c) processing the identified plurality of table bounds, using table parsing techniques, to generate the plurality of structured tables with the associated table captions;
process the identified plurality of structured tables with the associated table captions along with a plurality of text chunks, using extraction prompts generated via a chemical composition schema, to generate a plurality of chemical composition instances; and
reconcile the plurality of chemical composition instances into a unified knowledge graph.

8. The system as claimed in claim 7, the unified knowledge graph provides one or more chemical composition instances among the plurality of chemical composition instances based on a received user query.

9. The system as claimed in claim 7, wherein the table caption for the identified plurality of table bounds is obtained by:
a) computing a table dimension further comprising a top-left table dimension and a bottom-right table dimension for each table bound of the identified plurality of table bounds, using a table height of the table bound, a table width of the table bound, and the middle point of table bounds;
b) computing a surrounding text bound height as n% of the height of the table bound;.
c) computing a surrounding text bound further comprising a top-left surrounding text bound and a bottom-right surrounding text bound, at top of the table bound, using the table dimension, and the surrounding text bound height;
d) capturing a surrounding text in the surrounding text bounds, using a text parsing technique; and
e) identifying the table caption, by iteratively traversing each line in the surrounding text, using a Regex technique, wherein if the table caption is not found at the top of the table bound, the surrounding text bound is recomputed at the bottom of the table bound using the table dimension, and the surrounding text bound height, and wherein the table caption is identified by iteratively traversing each line in the surrounding text, using the Regex technique.

10. The system as claimed in claim 7, wherein the extraction prompts generated using the chemical composition schema comprises the molecular extraction prompt and the composition instance extraction prompt.

11. The system as claimed in claim 7, wherein the chemical composition schema is generated through prompting one of a plurality of Generative Pre-Trained Transformers (GPT) models using a chemical domain guided input prompt, and wherein the generated chemical composition schema is postprocessed based on the at least one target property of interest among the plurality of target properties.

12. The system as claimed in claim 7, the plurality of chemical composition instances is generated by:
a) feeding the plurality of structured tables with the associated table captions, the plurality of text chunks from the plurality of documents, a molecular extraction prompt, to one of the plurality of GPT models, wherein the one of the plurality of GPT model identifies a plurality of molecules present in the plurality of structured tables with table captions and in the plurality of text chunks;
b) applying a sequential parsing technique on the identified plurality of molecules to generate a plurality of groups of molecules; and
c) sequentially feeding each group amongst the plurality of groups of molecules, the plurality of structured tables with table captions, the plurality of text chunks, the chemical composition schema, and the composition instance extraction prompt, to one of the plurality of GPT models, to generate the plurality of chemical composition instances with a plurality of target molecular property values associated with the plurality of target properties.

13. One or more non-transitory machine-readable information storage mediums comprising one or more instructions which when executed by one or more hardware processors cause:
receiving a plurality of documents based on at least one target property of interest amongst a plurality of target properties associated with a plurality of chemical entities, from a plurality of data sources, wherein the plurality of data sources comprises at least one of (i) a plurality of patent documents, (ii) a plurality of technical publications, and (iii) a plurality of chemical databases;
identifying a plurality of structured tables with associated table captions, from each of the plurality of documents by:
a) identifying a plurality of table bounds in the plurality of documents using one or more deep learning-based boundary detection techniques;
b) obtaining a table caption for each of the identified plurality of table bounds; and
c) processing the identified plurality of table bounds, using table parsing techniques, to generate the plurality of structured tables with the associated table captions;
processing the identified plurality of structured tables with the associated table captions along with a plurality of text chunks, using extraction prompts generated via a chemical composition schema, to generate a plurality of chemical composition instances; and
reconciling the plurality of chemical composition instances into a unified knowledge graph.

14. The one or more non-transitory machine-readable information storage mediums as claimed in claim 13, the unified knowledge graph provides one or more chemical composition instances among the plurality of chemical composition instances based on a received user query.

15. The one or more non-transitory machine-readable information storage mediums as claimed in claim 13, wherein the table caption for each of the identified plurality of table bounds is obtained by:
a) computing a table dimension further comprising a top-left table dimension and a bottom-right table dimension for each of the table bound of the identified plurality of table bounds, using a table height of the table bound, a table width of the table bound, and a middle point of the table bound;
b) computing a surrounding text bound height as **n%** of the height of the table bound;.
c) computing a surrounding text bound further comprising a top-left surrounding text bound and a bottom-right surrounding text bound, at top of the table bound, using the table dimension, and the surrounding text bound height;
d) capturing a surrounding text in the surrounding text bounds, using a text parsing technique; and
e) identifying the table caption, by iteratively traversing each line in the surrounding text, using a Regex technique, wherein if the table caption is not found at the top of the table bound, the surrounding text bound is recomputed at the bottom of the table bound using the table dimension, and the surrounding text bound height, and wherein the table caption is identified by iteratively traversing each line in the surrounding text, using the Regex technique.
